(19) Europäisches Patentamt
European Patent Office
Office européen des brevets

(11) Veröffentlichungsnummer: **0 089 693**
**B1**

(12) # EUROPÄISCHE PATENTSCHRIFT

(45) Veröffentlichungstag der Patentschrift: **06.11.85**

(51) Int. Cl.[4]: **C 07 J 41/00,** C 07 C 127/15, A 61 K 31/22, A 61 K 31/56, A 61 K 37/02

(21) Anmeldenummer: **83102894.9**

(22) Anmeldetag: **23.03.83**

(54) **Steroidester von N-(2-Halogenethyl)-N-nitroso-carbamoyl-aminosäuren und deren Peptiden, sowie Verfahren zu deren Herstellung.**

(30) Priorität: **23.03.82 DE 3210637**

(43) Veröffentlichungstag der Anmeldung: **28.09.83 Patentblatt 83/39**

(45) Bekanntmachung des Hinweises auf die Patenterteilung: **06.11.85 Patentblatt 85/45**

(84) Benannte Vertragsstaaten: **AT BE CH DE FR GB IT LI LU NL SE**

(56) Entgegenhaltungen:
**FR - A - 2 220 261**
**GB - A - 2 028 336**

**JOURNAL OF MEDICINAL CHEMISTRY, Band 22, nr. 2, Nr. 2, Februar 1979, Seiten 200-202, American Chemical Society, Washington, DC, USA HING-YAT P. LAM et al.: "Synthesis of steroidal nitrosoureas with antitumor activity"**
**CHEMICAL ABSTRACTS, Band 96, Nr. 9, 1. März 1982, Seite 654, Nr. 69391*c*, Columbus, Ohio, USA W.C. TANG et al.: "Synthesis of potentially antineoplastic derivatives of N-(N-(2-chloroethyl)-N-nitrocarbamoyl)amino acids"**
**Wall u. Mitarbeiter - *Journal of Medicinal Chemistry* - Sept. 1969 Seite 812-2) Niculescu-Duval, Journal of Medicinal Chemistry, März 1967, *Seite 172***

(73) Patentinhaber: **Stiftung Deutsches Krebsforschungszentrum, Im Neuenheimer Feld 280, D-6900 Heidelberg 1 (DE)**

(72) Erfinder: **Eisenbrand, Gerhard, Dr., Banngartenstrasse 19 a, D-6902 Sandhausen (DE)**
Erfinder: **Schreiber, Joachim, Dr., Mittlere Kirchgasse 2, D-6900 Heidelberg 1 (DE)**

(74) Vertreter: **Deufel, Paul, Dr., Patentanwälte Müller-Boré, Deufel, Schön, Hertel Lewald, Otto Isartorplatz 6 Postfach 26 02 47, D-8000 München 26 (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

EP 0 089 693 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Gegenstand der vorliegenden Patentanmeldung sind Steroid-N-(2-halogenethyl)-N-nitroso-carbamoyl-aminosäuren bzw. Peptidester der allgemeinen Formel:

$$Hal-CH_2-CH_2-N(NO)-CO-NH-\underset{R_1}{\underset{|}{CH}}-CO-(NH-\underset{R_2}{\underset{|}{CH}}-CO)_n-OR_3$$

worin $R_1$ und $R_2$, die gleich oder verschieden sein können, den Rest einer Aminosäure ab dem betaständigen C-Atom (falls vorhanden) bedeuten, $R_3$ der Rest eines Steroids oder eines pharmakologisch ähnlich wirksamen Stilben-Derivates mit mindestens einer sterisch nicht gehinderten OH-Gruppe ist, n eine Zahl von 0-5 und Hal Chlor oder Fluor bedeutet.

N-(2-Chlorethyl)-N-nitroso-harnstoffe («CNU»), z.B. 1,3-bis-(2-Chlorethyl)-1-nitroso-harnstoff (BCNU), 1-(2-Chlorethyl)-3-cyclohexyl-1-nitroso-harnstoff (CCNU) und 1-(2-Chlorethyl)-3-(4-methyl) cyclohexyl-1-nitroso-harnstoff (MeCCNU) sind wichtige antineoplastische Chemotherapeutika, auch in klinischer Hinsicht. (Nitrosoureas in Cancer Treatment; Editors: B. Serrou, P.S. Schein and J.-L. Imbach; Elsevier/North-Holland Biomedicals Press, 1981 – Nitrosoureas: current status and new developments, Academic Press, New York, 1981).

Neben der therapeutischen Wirksamkeit weisen diese Substanzen aber auch eine langdauernde kumulative Toxizität auf (Eisenbrand et al. in: Nitrosoureas in Cancer Treatment, Elsevier 1981).

Um Substanzen mit besserem therapeutischen Index zu erhalten, wurden in neuerer Zeit Nitrosoharnstoffe mit verschiedenen Substituenten (z.B. Zucker, Peptide, DNS-Basen) synthetisiert und experimentell getestet.

Es hat sich gezeigt, dass eine Reihe von Tumoren Hormonrezeptoren enthalten bzw. hormonabhängig sind. Schon mehrfach ist versucht worden, die Chemotherapie von solchen Tumoren dadurch zu verbessern, dass beispielsweise Alkylantien chemisch an Hormone gebunden wurden, um die Rezeptoraffinität der Hormone auszunutzen zum gezielten Transport des cytostatischen Alkylans in das Tumorgewebe. Substanzen solcher Art sind beispielsweise Prednimustin®, ein Ester aus Prednisolon und dem Alkylans Chlorambucil (einem Phenyl-buttersäure-N-Lost-Derivat) oder Estracyt®, ein N,N-bis(2-chlorethyl)-3-Carbamat des Östradiol-17-β-Phosphats. (Cancer Chemotherapy; Edited by H.M. Pinedo, Excerpta Medica, Amsterdam-Oxford, 1979 und 1980).

Im Gegensatz zu diesen direkt alkylierenden N-Lost-Derivaten handelt es sich im vorliegenden Fall um 2-Halogen-alkylnitrosoharnstoff-Derivate, die durch ihren Zerfall in vivo erst ein quervernetzendes Alkylans freisetzen. Die CNU-Gruppierung ist somit chemisch und biologisch anders zu bewerten als die N-Lost-Gruppierung. In der Regel haben die CNU-Derivate eine grössere therapeutische Breite als die N-Lost-Derivate. Bei den hier beschriebenen Verbindungen ist ein Steroidmolekül oder ein pharmakologisch ähnlich wirkendes Stilbenderivat über eine Ester-Bindung mit einer N-(2-Halogenethyl)-N-nitroso-carbamoyl-aminosäure bzw. mit einer Peptidkette verbunden, deren terminale Aminosäuren die N-(2-Halogenethyl)-N-nitroso-carbamoyl-Gruppe trägt. Der Ausdruck Steroid umfasst hier auch pharmakologisch ähnlich wirkende Stilbenderivate mit mindestens einer sterisch nicht gehinderten OH-Gruppe. Die Steroide sind vorzugsweise solche der Östran-, Androstan- oder Pregnan-Reihe oder Corticosteroide. Die zur Veresterung herangezogenen OH-Gruppen befinden sich vorzugsweise in Stellung 3, 6, 7, 15, 16, 17 und 21 Stellung des Pregnan-Gerüsts. Zusätzlich vorhandene OH-Gruppen können frei oder auch verethert (z.B. mit Methyl oder Ethyl) oder verestert (z.B. mit Acetat oder Propenat) sein.

Die Stilbenderivate sind vorzugsweise solche der folgenden Strukturen.

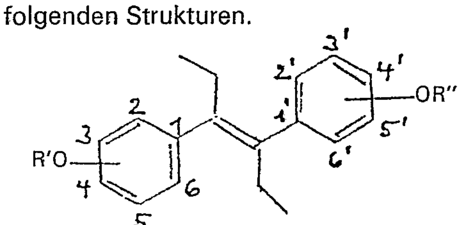

oder 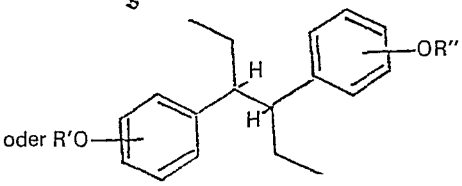

mit R'=Hal−CH₂−CH₂−N(NO)−CO−

$$NH-\underset{R_1}{\underset{|}{CH}}-\overset{O}{\overset{\|}{C}}-(NH-\underset{R_2}{\underset{|}{CH}}-CO)_n-$$

R' ist also der Rest zur Ergänzung des Chlorethylnitroso-harnstoffes, wobei R' und R'' die vorher angegebenen Bedeutungen besitzen, also Reste von Aminosäuren sind. R'' = H oder R' oder niedriges Alkyl, insbesondere Methyl oder Ethyl oder Acyl, insbesondere Acetyl oder Benzoyl. Die OR' bzw. OR''-Substituenten sitzen an gleichen oder verschiedenen Stellungen der Benzol-Ringe, insbesondere in Stellung 3, 3'; 4, 4' und 3, 4'. Die Ethylen-Doppelbindung des Stilben-Körpers kann auch hydriert sein.

Zur Herstellung dieser Verbindungen wird eine CNU-Aminosäure, ein CNU-Di-, Tri- oder -Oligopeptid (bis Hexapeptid) (synthetisiert nach Tang, W., Eisenbrand, G., Arch. Pharm. 314, 910-917, (1981)) und Patentanmeldung P 31 34 923.4 in eine aktivierte Form gebracht, wie z.B. das Imidazolid oder ein gemischtes Säureanhydrid (z.B. das para-Toluolsulfonsäure) und mit einem Steroidalkohol umgesetzt, der mindestens eine sterisch nicht gehinderte OH-Gruppe trägt (z.B. reagiert die gehinderte 11-OH-Gruppe nicht). Die CNU-Säurekomponente lässt sich auch direkt mit dem Steroidalkohol unter Zuhilfenahme von Kondensationsmitteln wie z.B. Dicyclohexylcarbodiimid verestern. Diese Reaktionen können mit und ohne Acylierungskatalysator wie z.B. 4-Dimethylaminopyridin durchgeführt werden. Sind mehrere OH-Gruppen im Steroid enthalten, so reagieren die reakivsten bevorzugt. Des weiteren lässt sich dieser Verbindungstyp herstellen, indem man von einem Steroid-Aminosäureester ausgeht und an diesen die CNU-Funktion anhängt, z.B. durch Umsetzung mit N-(2-Chlorethyl)-N-nitroso-carbamoyl-azid. Schliesslich kann dieser Steroid-Aminosäureester auch mit CNU-Aminosäuren oder CNU-Di-, Tri oder Oligopeptiden (bis Pentapeptiden) unter Zuhilfenahme von milden Kondensationsreagenzien wie z.B. Dicyclohexylcarbodiimid zum gewünschten Produkt umgesetzt werden.

Die Reaktionen lassen sich wie folgt darstellen:

A. $Hal-CH_2-CH_2-N(NO)CO-NH-CHR_1-CO-(NH-CHR_2-CO)_n-OH$

Aktivierung

$Hal-CH_2-CH_2-N(NO)CO-NH-CHR_1-CO-(NH-CHR_2-CO)_n-X$

X=z.B.- 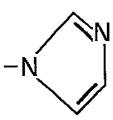 ; $-O-SO_2-$ 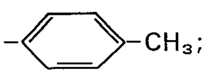 ;

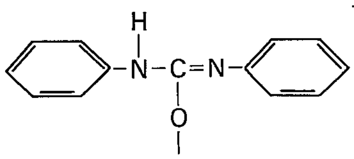

+Steroid −OH
−HX

$Hal-CH_2-CH_2-N(NO)CO-NH-CHR_1-CO-(NH-CHR_2-CO)_n-O-Steroid$

$R_1$, $R_2$, n und Hal haben hier und im Schema B und C die im Anspruch 1 angegebenen Bedeutungen. X ist eine reaktive einwertige Gruppe.

B. $Steroid-O-(CO-CHR_2-NH)_n-CO-CHR_1-NH_2$
$+ X-CO-N(NO)-CH_2-CH_2-Hal$

X = z.B. $N_3$; 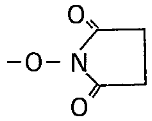 ;

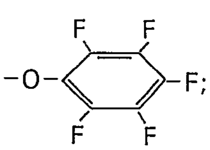 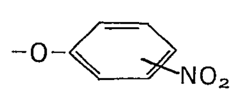

−HX ortho u. para

$Steroid-O-(CO-CHR_2-NH)_n-CO-CHR_1-NH-CO-N(NO)-CH_2-CH_2-Hal$

C. $Steroid-O-(CO-CHR_2-NH)_k-CO-CHR_2-NH_2$
$+HO(OC-CHR_2-NH)_1-CO-CHR_1-NH-CO-N(NO)-CH_2-CH_2-Hal$

Dicyclohexylcarbodiimid

$Steroid-O-(CO-CHR_2-NH)_n-CO-CHR_1-NH-CO-N(NO)-CH_2-CH_2-Hal$

mit k = 0 bis 3
l = 0 bis 4
n = 0 bis 5

Die Reste $R_1$ und $R_2$ sind Reste von Aminosäuren ab dem beta-ständigen Kohlenstoffatom. Im Falle von Glycin fehlt dieses, so dass diese Reste Wasserstoff bedeuten. Als weitere Reste seien genannt:

$-CH_3$, $-CH(CH_3)_2$, $-CH_2-SH$, $-CH_2-OH$, $-CH_2-CH(CH_3)_2$, $-CH(CH_3)C_2H_5$,

$-CH_2-$ 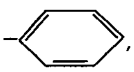, $-CH_2-CH_2-S-CH_3$,

$-CH_2-S-S-CH_2-CH(NH_2)-CO_2H$,
$-CH_2-S-S-CH_2-CH(CO_2H)-NH-CO-N(NO)-CH_2-CH_2-Hal$,
$-CH_2-S-S-CH_2-CH(CO_2R_3)-NH-CO-N(NO)-CH_2-CH_2-Hal$,
$-CH_2-CONH_2$, $-(CH_2)_2-CO-NH_2$,

$-CH_2-$ 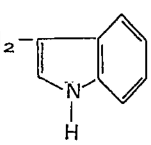

$R_1$ und $R_2$ können gleich oder verschieden sein, wobei bei n 1 die Reste $R_2$ auch untereinander gleich oder verschieden sein können.

$R_3$ bedeutet den Rest eines Steroids, insbesondere solche, die den Klassen der Androgene, Östrogene, Gestagene und Corticosteroide zuzuordnen sind, kann aber auch den Rest eines pharmakologisch ähnlich wirksamen Stilben-Derivats bedeuten, wie dem Diethylstilboestrol.

Der Begriff Hal bedeutet hier Fluor oder Chlor.

Bei der Verfahrensvariante B kann die reaktive Gruppe X insbesondere $N_3$, $O-C_6F_5$,

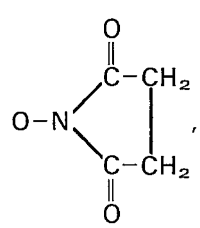 ,

ortho $O-C_6H_4(NO_2)$, para $O-C_6H_4(NO_2)$, bedeuten.

Bei der Verfahrensvariante wird Dicyclohexylcarbodiimid als Kondensationsmittel bevorzugt. Hier besteht auch die Alternativmöglichkeit, eine Umsetzung im wasserhaltigen System unter Einsatz wasserlöslicher Carbodiimide durchzuführen, wie beispielsweise dem 1-Cyclohexyl-3-(2-morpholino-ethyl)-carbodiimid-metho-4-toluolsulfonat oder dem 1-Ethyl-3-(3-dimethylaminopropyl)-carbodiimid-hydrochlorid. Im Falle wasserlöslicher Carbodiimide können also auch wasserhaltige Lösungsmittelsysteme verwendet werden.

Die folgenden Beispiele erläutern die Erfindung:

*Beispiel 1*

n-(2-Chlorethyl)-N-nitroso-carbamoyl-L-alanin-Cortison-21-ester

a. 1,7 g N,N-Carbonyldiimidazol werden in 50 ml absolutem Tetrahydrofuran (THF) mit 2,3 g CNU-L-Alanin versetzt, wobei unter starker Gasentwicklung das gewünschte Imidazolid entsteht. Zur vollständigen Umsetzung lässt man 1 h bei Raumtemperatur (RT) rühren. Dann gibt man 3,6 g Cortison zu und rührt 6 h bei RT. Nun wird das THF am Rotationsverdampfer abgezogen und der Rückstand in 500 ml Essigester aufgenommen. Es wird mit Eiswasser, eiskalter $NaHCO_3$-Lösung, eiskalter $NaHSO_4$-Lösung, Eiswasser und gesättigter NaCl-Lösung ausgeschüttelt und der gelbe Essigester-Extrakt über $Na_2SO_4$ getrocknet.

Nach Chromatographie über Kieselgel erhält man 4,2 g (75%) n-(2-Chlorethyl)-N-nitroso-carbamoyl-L-alanin-Cortison-21-ester. Umkristallisieren durch Aufnehmen im Dichlormethan oder Essigester und langsame Zugabe von n-Pentan und dann Abkühlen im Tiefkühlschrank ergibt hellgelbe Kristalle.

Fp.: 169° C (Zersetzung!)

*Elementaranalyse*

| | | | | |
|---|---|---|---|---|
| Ber.: | C 57,29 | H 6,41 | Cl 6,26 | N 7,42% |
| Gef.: | C 57,43 | H 6,62 | Cl 6,20 | N 7,32% |

Das $^{13}$C-NMR-Spektrum steht mit der angegebenen Struktur im Einklang.

Ebenso wurden hergestellt:

b. N-(2-Chlorethyl)-N-nitroso-carbamoyl-L-alanin-pregnenolon-Ester. Ausbeute 50%

hellgelbe Kristalle, Fp.: 158° C (Zers.) (aus Dichlomethan/n-Pentan wie Bsp. 1a)

*Elementaranalyse*

| | | | | |
|---|---|---|---|---|
| Ber.: | C 62,12 | H 7,72 | Cl 6,79 | N 8,05% |
| Gef.: | C 61,94 | H 7,98 | Cl 7,00 | N 7,98% |

c. N-(2-Chlorethyl)-N-nitroso-carbamoyl-L-alanin-östron-ester, Ausbeute: 60%

hellgelbe Kristalle, Fp.: 158° C (Zers.) (Umkristallisieren wie Bsp. 1a)

*Elementaranalyse*

| | | | | |
|---|---|---|---|---|
| Ber.: | C 60,56 | H 6,35 | N 8,83 | Cl 7,45% |
| Gef.: | C 60,76 | H 6,58 | N 8,63 | Cl 7,59% |

d. N-(2-Chlorethyl)-N-nitroso-carbamoyl-L-alanin-östradiol-3-ester, Ausbeute: 60%

hellgelbe Kristalle, Fp.: 140° C (Zers.) (Umkristallisieren wie Bsp. 1a)

*Elementaranalyse*

| | | | | |
|---|---|---|---|---|
| Ber.: | C 60,31 | H 6,75 | Cl 7,42 | N 8,79% |
| Gef.: | C 60,42 | H 7,05 | Cl 7,50 | N 9,03% |

Das $^{13}$C-NMR-Spektrum steht mit der angegebenen Struktur in Einklang.

e. N-(2-Chlorethyl)-N-nitroso-carbamoyl-L-alanin-prednisolon-21-ester, Ausbeute: 70%

hellgelbe Kristalle, Fp.: 168° C (Zers.) nach Aufnehmen in THF, Zugabe von Essigester und Kühlen

*Elementaranalyse*

| | | | | |
|---|---|---|---|---|
| Ber.: | C 57,29 | H 6,41 | N 7,42% | |
| Gef.: | C 57,07 | H 6,51 | N 7,38 | Cl 6,35% |

Das $^{13}$C-NMR-Spektrum steht mit der angegebenen Struktur in Einklang.

f. N-(2-Chlorethyl)-N-nitroso-carbamoyl-L-alanin-cis-androsteronester, Ausbeute 60%

hellgelbe Nadeln, Fp.: 114° C (Zers.)

*Elementaranalyse*

| | | | | |
|---|---|---|---|---|
| Ber.: | C 60,53 | H 7,72 | Cl 7,15 | N 8,47% |
| Gef.: | C 60,78 | H 7,91 | Cl 6,98 | N 8,50% |

Das $^{13}$C-Spektrum bestätigt die angegebene Struktur.

g. N-(2-Chlorethyl)-N-nitroso-carbamoyl-L-alanin-desoxycorticosteron-ester, Ausbeute 70%

hellgelbe Nadeln, Fp.: 107° C (Zers.)

*Elementaranalyse*

| | | | | |
|---|---|---|---|---|
| Ber.: | C 60,50 | H 7,15 | Cl 6,61 | N 7,84% |
| Gef.: | C 60,52 | H 7,05 | Cl 6,81 | N 8,05% |

h. N-(2-Chlorethyl)-N-nitroso-carbamoyl-L-alanin-dihydrotestosteron-17β-ester, Ausbeute: 70%

hellgelbe Nadeln, Fp.: 130° C (Zers.)

*Elementaranalyse*

| | | | | |
|---|---|---|---|---|
| Ber.: | C 60,53 | H 7,72 | Cl 7,15 | N 8,47% |
| Gef.: | C 60,67 | H 7,82 | Cl 7,32 | N 8,39% |

i. N-(2-Chlorethyl)-N-nitroso-carbamoyl-L-alanyl-L-alanin-prednisolon-21-ester, Ausbeute: 50%

hellgelbe Kristalle, Fp.: 130° C

*Elementaranalyse*

| | | | |
|---|---|---|---|
| Ber.: | C 56,56 | H 6,49 | N 8,79% |
| Gef.: | C 56,26 | H 7,12 | N 8,41% |

Analog den vorhergehenden Beispielen kann statt des Steroidalkohols ein Stilbenderivat mit phenolischer OH-Gruppe umgesetzt werden.

Wird CNU-Alanin/Kupplungsreagens im Überschuss eingesetzt, so entsteht mit Diethylstilböstrol der Diester; bei CNU-Alanin/Kupplungsreagens im Unterschuss entsteht mit Diethylstilböstrol der Monoester bevorzugt.

k. N-(2-Chlorethyl)-N-nitroso-carbamoyl-L-alanin-diethylstilböstrol-diester

Ausbeute: 70%, hellgelbe Kristalle Fp.: 102° C (Zersetzung)

*Elementaranalyse*

| | | | | |
|---|---|---|---|---|
| Ber.: | C 53,02 | H 5,34 | Cl 10,43 | N 12,37% |
| Gef.: | C 52,98 | H 5,05 | Cl 10,35 | N 12,08% |

I. N-(2-Chlorethyl)-N-nitroso-carbamoyl-L-alanin-diethylstilböstrol-monoester

Ausbeute: 50%, hellgelbe Kristalle, Fp: 122° C

*Elementaranalyse*

| | | | | |
|---|---|---|---|---|
| Ber.: | C 60,82 | H 5,98 | Cl 7,48 | N 8,87% |
| Gef.: | C 60,60 | H 6,10 | Cl 7,31 | N 9,04% |

*Beispiel 2*

N-(2-Chlorethyl)-N-nitroso-carbamoyl-L-alanin-cortison-21-ester

1,2 g CNU-Alanin werden in 50 ml Pyridin gelöst. Bei 0° C werden 1 g p-Toluosulfonylchlorid zugesetzt. Es wird 15 min bei 0° C gerührt und dann 1,8 g Cortison zugegeben. Man lässt 2 h bei 0° C und weitere 5 h bei RT rühren. Dann wird die Reaktionsmischung auf ca. 1 l Eis-Wasser gegossen. Der Niederschlag wird abgenutscht, in 200 ml Essigester aufgenommen und mit eiskalter $NaHSC_4$-Lösung; Eis-$H_2O$; eiskalter $NaHCO_3$-Lösung; Eis-$H_2O$ und schliesslich mit gesättigter Kochsalz-Lösung ausgeschüttelt, über $Na_2SO_4$ getrocknet und über Kieselgel chromatographiert.

Ausbeute 50%

*Beispiel 3*

N-(2-Chlorethyl)-N-nitroso-carbamoyl-L-alanin-Östron-ester

1,2 g CNU-Alanin und 1,3 g Östron werden in 30 ml abs. Tetrahydrofuran gelöst. Bei 0° C wird 1,1 g Dicyclohexylcarbodiimid zugegeben. Nach etwa 5 min setzt starke Trübung ein und es entsteht ein weisser Niederschlag. Nach 2 h bei 0° C wird noch 5 h bei RT gerührt und der Niederschlag dann abfiltriert. Das Filtrat wird am Rotationsverdampfer eingeengt, in 200 ml Essig-ester aufgenommen und mit eiskalter $NaHCO_3$-Lösung, Eis-$H_2O$ und gesättigter Kochsalz-Lösung ausgeschüttelt. Nach der Trocknung über $Na_2SO_4$ wird über Kieselgel chromatographiert.

Ausbeute: 50%

*Daten zur Toxizität der Verbindungen*

N-(2-Chlorethyl)-N-nitroso-carbamoyl-L-alanin-cortison-21-ester: $LD_{50}$: 220-280 mg/kg bei 90 Tagen Beobachtungszeit.

$LD_{50}$ von CNU-Alanin: 40-50 mg/kg

An diesem Beispiel lässt sich zeigen, dass die Verknüpfung von CNU-Alanin mit Cortison eine starke Verminderung der Toxizität (um etwa das dreifache) bewirkt.

Beim Therapie-Test am Modell der Ratten-Leukämie L 5222 (Ivankovic, Zeller, Leukämie L 5222 des Rattenstammes BD IX. Eine durch Äthylnitrosoharnstoff induzierte monozytärmyeloische, transplantierbare Form für zytochemische und chemotherapeutische Studien. Blut, 28; 288-292, 1974 und Zeller, Ivankovic, Schmähl, Cancer Research Vol. 35, 1168-1174, 1975) ist das Cortison-Konjugat gut aktiv. Bei 176 mg/kg liegt der Gesamtmedian (Wiederholungsversuch!) bei 65 Tagen mit 3 geheilten Tieren von 6, während bei CNU-Alanin in equimolarer Dosis mit Cortison bei medianer Überlebenszeit von 20 Tagen keine Heilungen erreicht werden.

Beim N-(2-Chlorethyl)-N-nitroso-carbamoyl-L-alanin-östronester betrug die optimale Dosis bisher 351 mg/kg. Dabei beträgt der Gesamtmedian an der L 5222 43 Tage. Dies bedeutet, dass die Toxizität (gemessen an tumortragenden Tieren!) noch geringer ist als beim Cortison-Derivat.

Beim N-(2-Chlorethyl)-N-nitroso-carbamoyl-L-alanin-Östradiol-3-ester betrug die optimale Dosis bisher 279 mg/kg bei einer medianen Überlebenszeit von 49 Tagen bei 2 Heilungen. Die Östrogen-verknüpften Verbindungen wurden einem Östrogen-rezeptortest (Kranzfelder, Schneider, von Angerer, Schönenberger, J. Cancer Research Chim. Oncol. 97, 167-186 (1980) unterzogen (Lebercytosol). Es ergaben sich für das Östron-Derivat eine Affinität von 4% derjenigen des Östradiols (100%) für den Parameter 50% Bindungshemmung des zugesetzten $^3H$-markierten Östradiols und von 16% beim Östradiol-Derivat (bei jeweils 190 bzw. 50 mol).

## Patentansprüche

1. Steroid-N-(2-halogenethyl)-N-nitroso-carbamoyl-aminosäure bzw. Peptidester der allgemeinen Formel:

$$Hal-CH_2-CH_2-N(NO)-CO-NH-\underset{\displaystyle R_1}{\underset{|}{CH}}-CO-(NH-\underset{\displaystyle R_2}{\underset{|}{CH}}-CO)_n-OR_3$$

worin $R_1$ und $R_2$, die gleich oder verschieden sein können, den Rest einer Aminosäure ab dem beta-ständigen C-Atom (falls vorhanden) bedeuten, $R_3$ der Rest eines Steroids oder eines pharmakologisch ähnlich wirksamen Stilben-Derivates mit mindestens einer sterisch nicht gehinderten OH-Gruppe ist, n eine Zahl von 0-5 und Hal Chlor oder Fluor bedeutet.

2. Verfahren zur Herstellung von N-(2-Halethyl)-N-nitroso-carbamoyl-aminosäure-Steroidestern nach Anspruch 1, dadurch gekennzeichnet, dass man N-(2-Hal-ethyl)-N-nitroso-carbamoylaminosäuren oder entsprechend substituierte Di-, Tri- oder Oligopeptide (bis Hexapeptide) in einem geeigneten Lösungsmittel in an sich bekannter Weise unter Mitwirkung eines geeigneten Aktivierungs- bzw. Kondensationsmittels, insbesondere N,N'-Carbonyl-diimidazol, Dicyclohexylcarbodiimid oder p-Toluolsulfonylchlorid/tertiäres Amin, mit Steroidalkoholen oder den entsprechenden Stilbenderivaten mit mindestens einer sterisch nicht gehinderten OH-Gruppe zu den entsprechenden Steroidestern umsetzt.

3. Verfahren zur Herstellung von N-(2-Halethyl)-N-nitroso-carbamoyl-aminosäure-Steroidestern nach Anspruch 1, dadurch gekennzeichnet, dass man Steroid-aminosäure-ester oder Di-, Tri-oder Oligopeptidester (bis Hexapeptide) von Steroiden oder den entsprechenden Stilbenderivaten mit N-(2-Hal-ethyl)-N-nitroso-carbamoylierungsmitteln der allgemeinen Formel Hal-$CH_2$-$CH_2$-N(NO)CO-X, worin X eine reaktive Gruppe bedeutet, in einem geeigneten Lösungsmittel umsetzt.

4. Verfahren zur Herstellung von N-(2-Hal-ethyl)-N-nitroso-carbamoyl-peptidestern von Steroiden nach Anspruch 1, dadurch gekennzeichnet, dass man Steroid-aminosäure-ester oder die entsprechenden Stilbenderivate mit N-(2-Hal-ethyl)-N-nitrosocarbamoyl-aminosäuren oder entsprechend substituierten Di-, Tri- und Oligopeptiden (bis Pentapeptide) in einem geeigneten Lösungsmittel unter Einwirkung eines geeigneten Kondensationsmittels, insbesondere N,N'-Carbonyl-diimidazols, Dicyclohexylcarbodiimid oder p-Toluosulfonyl-chlorid/tertiäres Amin, umsetzt.

5. Verfahren nach Anspruch 2 bis 4, dadurch gekennzeichnet, dass die Umsetzung der Komponenten unter zusätzlicher Einwirkung von Acylierungskatalysatoren, insbesondere 4-Dimethylaminopyridin, 4-Pyrrolidino-pyridin oder 1-Hydroxybenzotriazol erfolgt.

6. Verfahren nach Anspruch 2 bis 4, dadurch gekennzeichnet, dass die Umsetzung in Tetrahydrofuran, $CH_2Cl_2$, $CHCl_3$, Benzol, Toluol oder Pyridin erfolgt.

7. Verfahren nach Anspruch 2 bis 4, dadurch gekennzeichnet, dass die Umsetzung der Komponenten in einem Temperaturbereich von −10° C bis zum Siedepunkt des Lösungsmittels, höchstens aber bis zur Zerfallstemperatur des gewünschten Reaktionsproduktes bzw. der Reaktionspartner vorzugsweise bei Zimmertemperatur (RT) durchgeführt wird.

8. Verfahren nach Anspruch 2 bis 4, dadurch gekennzeichnet, dass als Steroidkomponente ein natürlich vorkommendes oder ein synthetisches Steroid eingesetzt wird.

## Revendications

1. Esters stéroïdiens ou peptidiques d'acide N-(2-halogénéthyl)-N-nitroso-carbamoyl-aminé, de formule générale

$$\text{Hal}-CH_2-CH_2-N(NO)-CO-NH-\underset{\displaystyle R_1}{\underset{|}{CH}}-CO-(NH-\underset{\displaystyle R_2}{\underset{|}{CH}}-CO)_n-OR_3$$

dans laquelle $R_1$ et $R_2$, qui peuvent être identiques ou différents, sont le résidu d'un acide aminé à partir de l'atome de carbone en position bêta (s'il existe), $R_3$ est le résidu d'un stéroïde ou d'un dérivé du stilbène ayant un effet pharmacologiquement analogue et ayant au moins un groupe OH ne présentant pas d'empêchement stérique, n est un nombre de 0 à 5 et Hal est le chlore ou le fluor.

2. Procédé pour la préparation d'esters stéroïdiens d'acide N-(2-Hal-éthyl)-N-nitroso-carbamoyl-aminé selon la revendication 1, caractérisé en ce qu'on fait réagir des acides N-(2-Hal-éthyl)-N-nitroso-carbamoyl-aminés ou des di-, tri- ou oligopeptides (jusqu'aux hexapeptides) substitués en conséquence, dans un solvant approprié, d'une manière connue en soi avec la collaboration d'un agent convenable d'activation ou de condensation, notamment le N,N'-carbonyldiimidazole, le dicyclohexylcarbodiimide ou le chlorure de p-toluènesulfonyle/amine tertiaire, sur des alcools stéroïdiens ou les dérivés correspondants du stilbène possédant au moins un groupe OH ne présentant pas d'empêchement stérique, pour donner les esters stéroïdiens correspondants.

3. Procédé pour la préparation d'esters stéroïdiens d'acides N-(2-Hal-éthyl)-N-nitroso-carbamoyl-aminés selon la revendication 1, caractérisé en ce qu'on fait réagir des esters stéroïdiens d'acides aminés ou des esters di-, tri- ou oligopeptidiques (jusqu'à hexapeptidiques) de stéroïdes ou de dérivés correspondants de stilbène, sur des agents de N-(2-Hal-éthyl)-N-nitroso-carbamoylation de formule générale Hal-$CH_2$-$CH_2$-N(NO)CO-X, dans laquelle X est un groupe réactif, dans un solvant approprié.

4. Procédé pour la préparation d'esters N-(2-Hal-éthyl)-N-nitroso-carbamoyl-peptidiques de stéroïdes selon la revendication 1, caractérisé en ce qu'on fait réagir des esters stéroïdiens d'acides aminés ou les dérivés correspondants du stilbène sur des acides N-(2-Hal-éthyl-N-nitroso-carbamoyl-aminés ou des di-, tri- et oligopeptides (jusqu'aux pentapeptides) substitués en conséquence, dans un solvant approprié, sous l'effet d'un agent de condensation approprié, notamment le N,N'-carbonyl-diimidazole, le dicyclohexyl-carbodiimide ou le chlorure de p-toluènesulfonyle/amine tertiaire.

5. Procédé selon les revendications 2 à 4, caractérisé en ce que la réaction des constituants s'effectue sous l'action supplémentaire de catalyseurs d'acylation, notamment la 4-diméthylaminopyridine, la 4-pyrrolidino-pyridine ou le 1-hydroxybenzotriazole.

6. Procédé selon les revendications 2 à 4, caractérisé en ce que la réaction a lieu dans du tétrahydrofuranne, du $CH_2Cl_2$, du $CHCl_3$, du benzène, du toluène ou de la pyridine.

7. Procédé selon les revendications 2 à 4, caractérisé en ce que la réaction des constituants se déroule dans un intervalle de température compris entre −10° C et le point d'ébullition du solvant, mais allant au plus jusqu'à la température de décomposition du produit de réaction recherché ou des produits participant à la réaction, de préférence à la température ambiante (RT).

8. Procédé selon les revendications 2 à 4, caractérisé en ce qu'on utilise en tant que constituant stéroïdien un stéroïde naturel ou synthétique.

## Claims

1. Steroid-N-(2-halogenoethyl)-N-nitroso-carbamoyl-amino-acid ester or peptide ester of the general formula:

$$Hal-CH_2-CH_2-N(NO)-CO-NH-\underset{R_1}{\underset{|}{CH}}-CO-(NH-\underset{R_2}{\underset{|}{CH}}-CO)_n-OR_3$$

in which $R_1$ and $R_2$, which can be identical or different, denote the radical of an amino acid starting from the C atom in the beta-position (if present), $R_3$ denotes the radical of a steroid or of a stilbene derivative, having a similar pharmacological action, with at least one sterically unhindered OH group, n denotes a number from 0-5 and Hal denotes chlorine or fluorine.

2. Process for the preparation of an N-(2-Hal-ethyl)-N-nitroso-carbamoyl-amino-acid steroid ester according to Claim 1, characterized in that N-(2-Hal-ethyl)-N-nitroso-carbamoyl-amino-acids or correspondingly substituted di-, tri- or oligo-peptides (up to hexapeptides) in a suitable solvent are reacted in a manner known per se, under the additional effect of a suitable activating or condensing agent, in particular N,N'-carbonyldiimidazole, dicyclohexylcarbodiimide or p-toluenesulphonyl chloride/tertiary amine, with steroid alcohols or the corresponding stilbene derivatives with at least one sterically unhindered OH group to give the corresponding steroid esters.

3. Process for the preparation of N-(2-Hal-ethyl)-N-nitroso-carbamoyl-amino-acid steroid esters according to Claim 1, characterized in that steroid amino acid esters or di-, tri- or oligopeptide esters (up to hexapeptides) of steroids or of the corresponding stilbene derivatives are reacted with N-(2-Hal-ethyl)-N-nitroso-carbamoylating agents of the general formula Hal-$CH_2$-$CH_2$-N(NO)CO-X, wherein X denotes a reactive group, in a suitable solvent.

4. Process for the preparation of N-(2-Hal-ethyl)-N-nitroso-carbamoyl-peptide esters of steroids according to Claim 1, characterized in that steroid amino-acid esters or the corresponding stilbene derivatives are reacted with N-(2-Hal-ethyl)-N-nitrosocarbamoyl-amino-acids or correspondingly substituted di-, tri- and oligopeptides (up to pentapeptides) in a suitable solvent under the action of a suitable condensing agent, in particular N,N'-carbonyl-diimidazole, dicyclohexylcarbodiimide or p-toluenesulphonyl chloride/tertiary amine.

5. Process according to Claims 2 to 4, characterized in that the reaction of the components is carried out under the additional action of acylation catalysts, in particular 4-dimethylaminopyridine, 4-pyrrolidino-pyridine or 1-hydroxy-benzotriazole.

6. Process according to Claims 2 to 4, characterized in that the reaction is carried out in tetrahydrofuran, $CH_2Cl_2$, $CHCl_3$, benzene, toluene or pyridine.

7. Process according to Claims 2 to 4, characterized in that the reaction of the components is carried out in a temperature range from −10° C up to the boiling point of the solvent, but at most up to the decomposition temperature of the desired reaction product or the reaction partners, preferably at room temperature (RT).

8. Process according to Claims 2 to 4, characterized in that a naturally occurring steroid or a synthetic steroid is employed as the steroid component.